# EUROPEAN PATENT SPECIFICATION

(11) **EP 4 101 474 B1**
(45) Date of publication and mention of the grant of the patent: **06.08.2025**
(21) Application number: 22176736.1
(22) Date of filing: 01.06.2022
(51) Int. Cl.: A61L 2/10, A61L 2/24, B64F 5/30, B64D 11/06

(54) **SYSTEMS FOR MOVEABLY SUPPORTING AN ULTRAVIOLET (UV) LIGHT LAMP WITHIN AN ENCLOSED SPACE**
SYSTEME ZUM BEWEGLICHEN HALTEN EINER UV-LAMPE IN EINEM GESCHLOSSENEN RAUM
SYSTÈMES DE SUPPORT AMOVIBLE D'UNE LAMPE À LUMIÈRE ULTRAVIOLETTE (UV) DANS UN ESPACE CLOS

(30) Priority: 09.06.2021 US 202163208636 P
(43) Date of publication of application: 14.12.2022
(73) Proprietor: The Boeing Company, Arlington, VA 22202 (US)
(72) Inventor: DACZKO, Mateus S., CHICAGO, 60606-2016 (US); BROWN, Douglas Alan, Arlington, VA 22202 (US)
(74) Representative: Plasseraud IP

(56) References cited:
- EP-A1- 2 767 154
- EP-A1- 3 293 118
- EP-A1- 3 922 555
- WO-A1-2023/158751
- CN-A- 113 827 747
- DE-A1- 102019 213 094
- GB-A- 2 391 799
- US-A1- 2016 136 314
- US-A1- 2017 290 935
- US-A1- 2022 313 856

## Description

### FIELD

Examples of the presently described subject matter generally relate to systems and methods for sanitizing surfaces within an enclosed space, such an internal cabin of a commercial aircraft, and more particularly to systems and methods for moveably supporting an ultraviolet (UV) light lamp within the enclosed space. Examples of the presently claimed subject matter relate to disinfecting systems for enclosed spaces.

### BACKGROUND

Vehicles such as commercial aircraft are used to transport passengers between various locations. Systems are currently being developed to disinfect or otherwise sanitize surfaces within aircraft, for example, that use ultraviolet (UV) light.

Typically, UV light disinfection is more effective when a UV light emitter is closer to a surface that is to be disinfected. However, in various settings, such as within the confined space of an internal cabin of a commercial aircraft, placing UV light emitters close to surfaces or areas to be disinfected may not be practical. As such, a UV light emitter may be spaced apart from an area or space that is to be disinfected at a relatively long distance. In such a scenario, the UV light emitter typically needs to be of sufficient power to effectively disinfect the target area or space. With increased distance to the target, the power typically needed for the UV light emitter is also generally increased. As can be appreciated, effective UV light disinfection within a particular setting may typically require an increased amount of time and power.

EP 3922555 A1, in accordance with its abstract, states a disinfection system that includes a base, a first arm mechanically coupled to the base at a first joint, a second arm mechanically coupled to the first arm at a second joint, and an emission module mechanically coupled to the second arm at a third joint. The emission module may include a third arm that includes one or more scanners configured to emit electromagnetic energy further configured to disinfect a first surface. The emission module may also include the one or more scanners, focusing lens, a lens frame, and a rotary actuator, wherein the rotary actuator is configured to align one of the one or more scanners with the focusing lens to produce a narrow electromagnetic beam, or position the focusing lens out of alignment with the one of the one or more scanners to produce a broad electromagnetic beam.

DE 10-2019/213094 A1, in accordance with its abstract, states a motor vehicle with a vehicle interior, comprising at least one first disinfection device with at least one first UV light source for disinfecting the air in the vehicle interior and at least one second disinfection device with at least one second UV light source for disinfecting at least one surface in the vehicle interior, the first disinfection device is arranged on an air-conditioning unit or fan of a ventilation device which supplies the vehicle interior with air or on an interior trim part underneath a reversibly movable cover, and the second disinfection device is arranged on an arm which can be moved in the vehicle interior.

US 2017/290935 A1, in accordance with its abstract, states systems and methods for disinfection of aircraft galleys and lavatories and other surfaces, using UV light sources and/or a disinfection unit powered by a fuel cell or other external power source.

US 2016/136314 A1, in accordance with its abstract, states sanitizing surfaces in a location related to aircraft with multiple rows of seats. A sanitization device includes a mobile body configured to travel along the aisle of the aircraft. An arm extends from the sanitization device laterally from the mobile body across the seats, and a source of UV radiation mounted on the sanitization device is directed across the seats exposing surfaces in the passenger area to UV radiation. There can be a unit hand operated, pushed down the aisle by hand, wings deployed and stowed by hand. There can be a plug-in version with long extension cords that a human can keep from tangling. The operator can be protected with skin and eye protective gear and there can be remote control by an operator standing nearby.

EP 3293118 A1, in accordance with its abstract, states an ultraviolet (UV) light sanitizing system and method to sanitize at least one surface within an enclosed space. The UV light sanitizing system includes a UV light assembly that is selectively moveable between a stowed position and a deployed position. The UV light assembly is stowed within a stowage chamber connected to the enclosed space in the stowed position. The UV light assembly deploys out of the stowage chamber and into the enclosed space in the deployed position.

### SUMMARY

A need exists for a UV light disinfecting system that can be quickly and easily positioned with respect to an area, space or the like that is to be disinfected. Further, a need exists for a UV light disinfecting system that can be moveably secured within a confined space, such as within an internal cabin of a vehicle. Also, a need exists for a UV light disinfecting system that utilize less power.

With those needs in mind, a disinfecting system for an enclosed space is defined, according to claim 1. Other embodiments of the disinfecting system are defined in the appended dependent claims 2 to 15.

Certain examples not forming part of the claimed subject matter, may provide a disinfecting method for an enclosed space. The disinfecting method includes moveably coupling an articulating arm to a structure within the enclosed space; coupling an ultraviolet (UV) lamp to the articulating arm, wherein the UV lamp is configured to emit UV light toward a disinfection zone within the enclosed space; and moving the UV lamp via the articulating arm relative to the disinfection zone.

Certain examples not forming part of the claimed subject matter, may provide an enclosed space including a structure, and a disinfecting system moveably coupled to the structure, as described herein.

### BRIEF DESCRIPTION OF THE DRAWINGS

Figure 1 illustrates a schematic block diagram of a disinfecting system within an enclosed space.
Figure 2 illustrates a perspective front view of an aircraft.
Figure 3A illustrates a top plan view of an internal cabin of an aircraft.
Figure 3B illustrates a top plan view of an internal cabin of an aircraft.
Figure 4 illustrates a perspective interior view of an internal cabin of an aircraft.
Figure 5 illustrates a lateral view of an example disinfecting system within an internal cabin of a vehicle, not forming part of the claimed subject matter.
Figure 6 illustrates a front view of the example disinfecting system within the internal cabin of Figure 5.
Figure 7 illustrates a lateral view of an example disinfecting system within an internal cabin of a vehicle, not forming part of the claimed subject matter.
Figure 8 illustrates a front view of the example disinfecting system within the internal cabin of Figure 7.
Figure 9 illustrates a lateral view of an example disinfecting system within an internal cabin of a vehicle, not forming part of the claimed subject matter.
Figure 10 illustrates a front view of the example disinfecting system within the internal cabin of Figure 9.
Figure 11 illustrates a front view of an example disinfecting system within an internal cabin of a vehicle, not forming part of the claimed subject matter.
Figure 12 illustrates a lateral view of a disinfecting system secured to a headrest, according to an example of the clamed subject matter.
Figure 13 illustrates a front perspective view of the disinfecting system of Figure 12.
Figure 14 illustrates an example flow chart of a disinfecting method, not forming part of the claimed subject matter.

### DETAILED DESCRIPTION

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

Certain examples of the presently described subject matter refer to a system and a method for positioning an ultraviolet (UV) lamp in close proximity to a target disinfection zone. The system and method include a mechanism that moves the UV lamp in relation to a structure, such as between a stowed position and a deployed position. In at least one example, the mechanism includes an articulating arm that moveably couples the UV lamp to a structure within an enclosed space, such as an internal cabin of a vehicle. The scope is determined with reference to the appended claims.

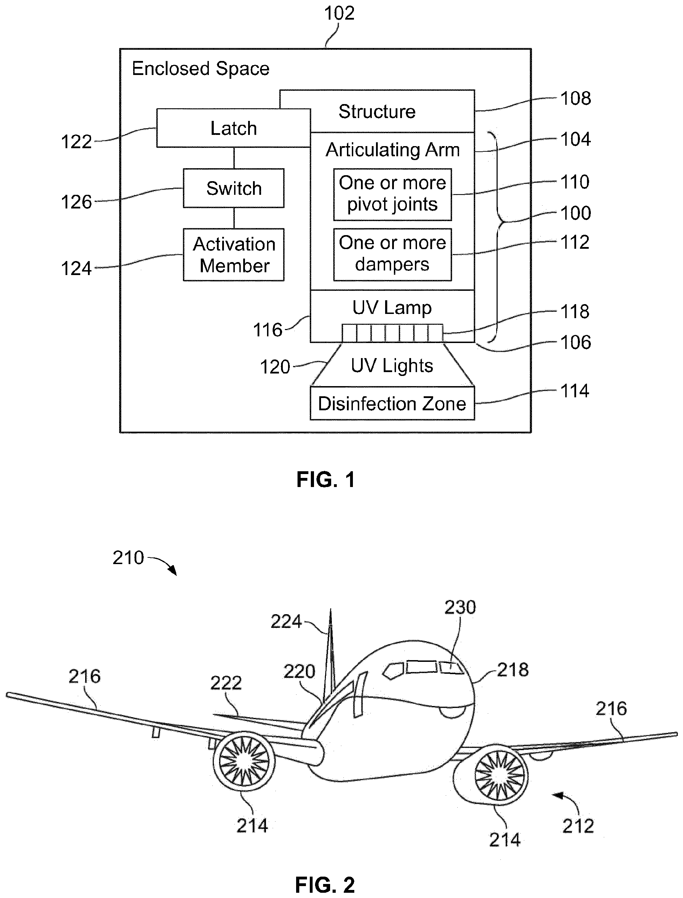

Figure 1 illustrates a schematic block diagram of a disinfecting system 100 within an enclosed space 102. In at least one example, the enclosed space 102 is an internal cabin of a vehicle, such as a commercial aircraft. In at least one other example, the enclosed space 102 is one or more rooms within a residential or commercial building. As an example, the enclosed space 102 can be an operating room within a hospital. In at least one other example, the enclosed space 102 is an open air venue, such as a stadium, concert space, and/or the like.

The disinfecting system 100 includes an articulating arm 104 that moveably secures an ultraviolet (UV) lamp 106 to a structure 108 within the enclosed space 102. In at least one example, the structure 108 is a ceiling within the enclosed space 102. As another example, the structure 108 is a panel within the enclosed space. For example, the structure 108 is a wall. In at least one example, the structure 108 is a spacer panel, a passenger service unit (PSU), overhead stowage bin assembly, or the like within an internal cabin of a vehicle. In at least one example, the structure 108 is a seat assembly within the enclosed space. For example, the structure 108 is a seat assembly within an internal cabin of a vehicle.

The articulating arm 104 includes one or more pivot joints 110 that allow the articulating arm 104 to pivot, rotate, or otherwise move. In at least one example, one or more dampers 112 are coupled to the one or more pivot joints 110 to control a rate of motion of the articulating arm 104. For example, the dampers 112 include or more brakes, clutches, servos, and/or the like. As an example, the dampers 112 can include a pneumatic damper. As another example, the dampers 112 can include a metal brake (such as an aluminum brake) coupled to a passive magnet. As another example, the dampers 112 can include electromagnetic brakes and/or clutches. Alternatively, the articulating arm 104 may not include any damper.

The articulating arm 104 allows the UV lamp 106 to be moved between various positions. For example, the articulating arm 104 is configured to allow the UV lamp 106 to be moved between a stowed position (such as recessed within a ceiling, spacer panel, PSU, headrest of a seat assembly, or the like), and a deployed position, in which a disinfection zone 114 is disinfected through operation of the UV lamp 106. The damper(s) 112 control a rate of motion of the articulating arm 104 as the UV lamp 106 is moved between different positions.

Optionally, the arm 104 can be configured to move through ways other than articulation. For example, the arm 104 can be pivotal, rotational, telescoping, and/or the like. As another example, the arm 104 can be fixed in position and not configured to move.

The UV lamp 106 includes a housing 116 having one or more UV light emitters 118, such as one or more bulbs, light emitting diodes (LEDs), and/or the like. The UV light emitters 118 are configured to emit UV light 120 onto or into the disinfection zone 114. In at least one example, the disinfection zone 114 includes an air space, such as a breathing space within the enclosed space 102. As another example, the disinfection zone 114 includes a surface of a structure (for example, the structure 108 and/or another structure) that is to be disinfected. The surface can be any physical surface within the enclosed space 102. For example, the surface can include walls, floors, ceilings, tables, countertops, seats, and/or the like within the enclosed space 102. In the example in which the enclosed space 102 is an internal cabin of a vehicle, the surfaces includes floors, walls, ceilings, passenger seats, counters, drawers, cabinets, and/or the like, such as within a passenger cabin, galleys, lavatories, and/or the like. As another example, the disinfection zone 114 includes an air space and a surface of a structure.

In at least one example, the UV lamp 106 emits the UV light 120 (such as through the UV light emitters 118) in a far UV light spectrum, such as at a wavelength of 222 nm, which neutralizes (such as kills) microbes (for example, viruses and bacteria), while posing no risk to humans. The UV lamp 106 may be used within the enclosed space 102 to decontaminate and kill pathogens. Optionally, the UV lamp 106 can be configured to emit the UV light 120 at other wavelengths, such as the UVC spectrum (for example, at a wavelength of 254 nm).

In at least one example, a latch 122 is coupled to the structure 108. The latch 122 also couples to the articulating arm 104. The latch 122 is configured to be selectively engaged or otherwise moved between a locked position and an unlocked position, so that the articulating arm 104 can be locked in position (such as in the stowed position), and unlocked (such as in the deployed position). For example, in the locked position, the latch 122 ensures that the articulating arm 104 is in the stowed position. In the unlocked position, the latch uncouples from the articulating arm 104, and the articulating arm 104 and the UV lamp 106 can moved into (for example, fall) into the deployed position, with the movement being controlled by the one or more dampers 112.

In at least one example, an activation member 124 that is configured to control the latch 122. For example, the activation member 124 is coupled to a switch 126, which is in turn coupled to the latch 122. For example, the activation member 124 is a button, dial, lever, or the like that is configured to be engaged by an individual. The activation member 124 can be disposed on or within the structure 108. For example, the activation member 124 can be in an armrest of a seat assembly, or secured to a wall, ceiling, or the like. As the activation member 124 is engaged by an individual, the switch 126 moves from the locked position to the unlocked position, thereby allowing the disinfecting system 100 to move into the deployed position. An individual may move the disinfecting system 100 back to the stowed position, at which the latch 122 automatically locks the disinfecting system 100 in place. Optionally, the disinfecting system 100 may not be coupled to the structure 108 through the latch 122. Also, optionally, the system may not include the activation member 124 and the switch 126.

As described herein, the disinfecting system 100 for the enclosed space 102 includes the articulating arm 104 moveably coupled to the structure 108 within the enclosed space 102. The UV lamp 106 is coupled to the articulating arm 104. The UV lamp 106 is configured to emit the UV light 120 toward (and subsequently onto and/or into) the disinfection zone 114 within the enclosed space 102. The articulating arm 104 allows the UV lamp 106 to move relative to the disinfection zone 114.

Figure 2 illustrates a perspective front view of an aircraft 210. The aircraft 210 includes a propulsion system 212 that includes engines 214, for example. Optionally, the propulsion system 212 may include more engines 214 than shown. The engines 214 are carried by wings 216 of the aircraft 210. In other examples, the engines 214 may be carried by a fuselage 218 and/or an empennage 220. The empennage 220 may also support horizontal stabilizers 222 and a vertical stabilizer 224.

The fuselage 218 of the aircraft 210 defines an internal cabin 230, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 230 is an example of an enclosed space 102, as shown in Figure 1.

Alternatively, instead of an aircraft, examples may be used with various other vehicles, such as automobiles, buses, locomotives and train cars, watercraft, and the like. Further, examples may be used with respect to fixed structures, such as commercial and residential buildings. In general, the sanitizing systems described herein may be used to sanitize various components, such as within enclosed spaces, outdoor spaces, and the like.

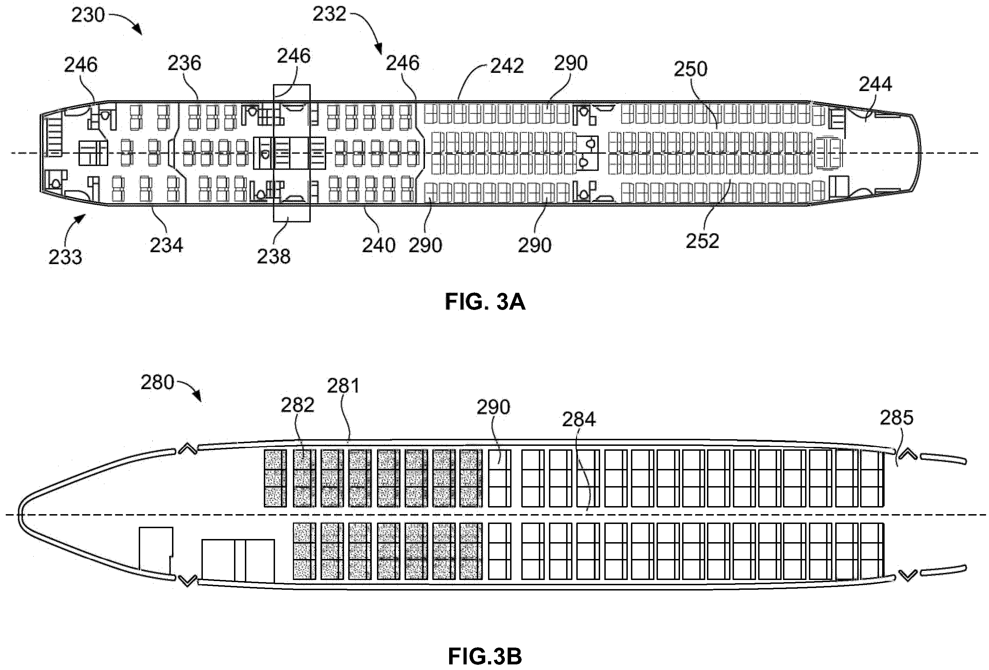

Figure 3A illustrates a top plan view of an internal cabin 230 of an aircraft. The internal cabin 230 may be within the fuselage 232 of the aircraft, such as the fuselage 218 of Figure 2. For example, one or more fuselage walls may define the internal cabin 230. The internal cabin 230 includes multiple areas, including a front section 233, a first-class section 234, a business class section 236, a front galley station 238, an expanded economy or coach section 240, a standard economy of coach section 242, and an aft section 244, which may include multiple lavatories and galley stations. It is to be understood that the internal cabin 230 may include more or less areas than shown. For example, the internal cabin 230 may not include a first-class section, and may include more or less galley stations than shown. Each of the sections may be separated by a cabin transition area 246, which may include class divider assemblies between aisles.

As shown in Figure 3A, the internal cabin 230 includes two aisles 250 and 252 that lead to the aft section 244. Optionally, the internal cabin 230 may have less or more aisles than shown. For example, the internal cabin 230 may include a single aisle that extends through the center of the internal cabin 230 that leads to the aft section 244.

The disinfecting system 100 shown in Figure 1 may be used to disinfect or otherwise sanitize various disinfection zones within the internal cabin 230, such as air space therein, passenger seats 290, monuments, stowage bin assemblies, components on and within lavatories, galley equipment and components, and/or the like.

Figure 3B illustrates a top plan view of an internal cabin 280 of an aircraft. The internal cabin 280 is an example of the internal cabin 230 shown in Figure 2. The internal cabin 280 may be within a fuselage 281 of the aircraft. For example, one or more fuselage walls may define the internal cabin 280. The internal cabin 280 includes multiple areas, including a main cabin 282 having passenger seats, and an aft section 285 behind the main cabin 282. It is to be understood that the internal cabin 280 may include more or less areas than shown.

The internal cabin 280 may include a single aisle 284 that leads to the aft section 285. The single aisle 284 may extend through the center of the internal cabin 280 that leads to the aft section 285. For example, the single aisle 284 may be coaxially aligned with a central longitudinal plane of the internal cabin 280.

The disinfecting system 100 shown in Figure 1 may be used to disinfect or otherwise sanitize various disinfection zones within the internal cabin 280, such as air space therein, passenger seats 290, monuments, stowage bin assemblies, components on and within lavatories, galley equipment and components, and/or the like.

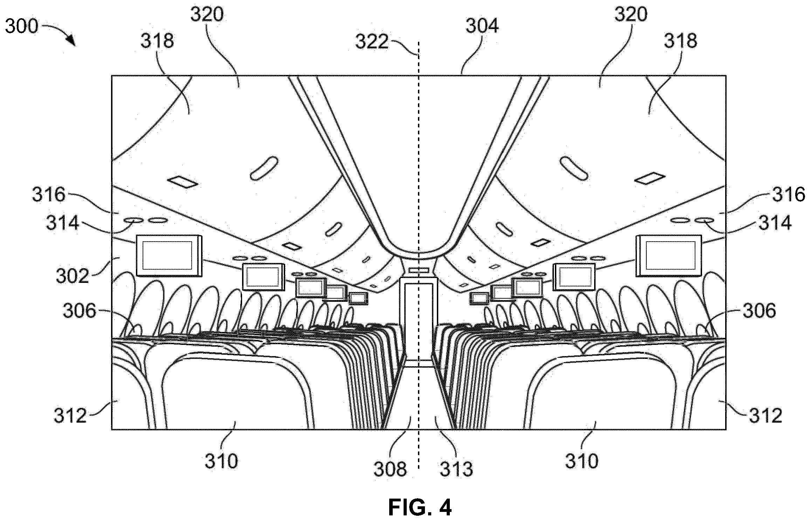

Figure 4 illustrates a perspective interior view of an internal cabin 300 of an aircraft. The internal cabin 300 includes outboard walls 302 connected to a ceiling 304. Windows 306 may be formed within the outboard walls 302. A floor 308 supports rows of seats 310. As shown in Figure 4, a row 312 may include two seats 310 on either side of an aisle 313. However, the row 312 may include more or less seats 310 than shown. Additionally, the internal cabin 300 may include more aisles than shown.

Passenger service units (PSUs) 314 are secured between an outboard wall 302 and the ceiling 304 on either side of the aisle 313. The PSUs 314 extend between a front end and rear end of the internal cabin 300. For example, a PSU 314 may be positioned over each seat 310 within a row 312. Each PSU 314 may include a housing 316 that generally contains vents, reading lights, an oxygen bag drop panel, an attendant request button, and other such controls over each seat 310 (or groups of seats) within a row 312.

Overhead stowage bin assemblies 318 are secured to the ceiling 304 and/or the outboard wall 302 above and inboard from the PSU 314 on either side of the aisle 313. The overhead stowage bin assemblies 318 are secured over the seats 310. The overhead stowage bin assemblies 318 extend between the front and rear end of the internal cabin 300. Each stowage bin assembly 318 may include a pivot bin or bucket 320 pivotally secured to a strongback. The overhead stowage bin assemblies 318 may be positioned above and inboard from lower surfaces of the PSUs 314. The overhead stowage bin assemblies 318 are configured to be pivoted open in order to receive passenger carry-on baggage and personal items, for example.

As used herein, the term "outboard" means a position that is further away from a central longitudinal plane 322 of the internal cabin 300 as compared to another component. The term "inboard" means a position that is closer to the central longitudinal plane 3622 of the internal cabin 300 as compared to another component. For example, a lower surface of a PSU 314 may be outboard in relation to a stowage bin assembly 318.

The disinfecting system 100 shown in Figure 1 may be used to disinfect or otherwise sanitize various disinfection zones within the internal cabin 300, such as air space therein, passenger seats 310, monuments, stowage bin assemblies, components on and within lavatories, galley equipment and components, and/or the like. The seats 310, the PSUs 314, spacer panels between the PSUs 314, the stowage bin assemblies 318, the outboard walls 302, the ceiling 304, and the like are examples of structures, such as the structure 108 shown in Figure 1, to which the disinfecting system 100 can be moveably secured.

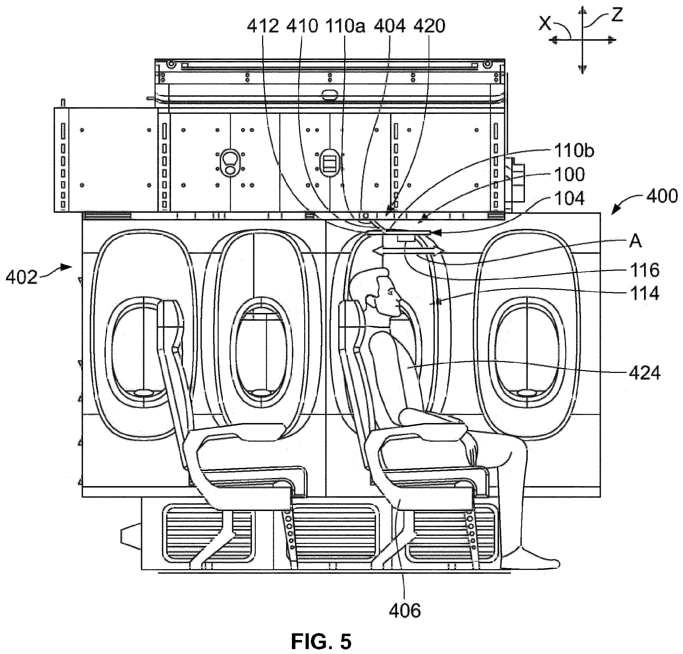

Figure 5 illustrates an example of a lateral view of the disinfecting system 100 within an internal cabin 400 of a vehicle 402 (such as a commercial aircraft). The internal cabin 400 is an example of the enclosed space 102 shown in Figure 1. The disinfecting system 100 is moveably coupled to a spacer panel 404 above a seat 406 within the internal cabin 400. The spacer panel 404 is an example of the structure 108 shown in Figure 1.

In at least one example, the disinfecting system 100 includes a plurality of arm segments that are configured to move relative to one another. The plurality of arm segments can be moveably coupled to each other through a plurality of pivot joints. Any of the examples described herein can include the plurality of arm segments. For example, the disinfecting system 100 includes the articulating arm 104, which includes a first arm segment 410 and a second arm segment 412. The first arm segment 410 moveably coupled to the spacer panel 404 through a first pivot joint 110a. The first arm segment 410 is coupled to the second arm segment 412 through a second pivot joint 110b. The articulating arm 104 is able to pivot, rotate, and/or otherwise move about the first pivot joint 110a and the second pivot joint 110b. The first pivot joint 110a and the second pivot joint 110b allow the articulating arm 104 to move between the deployed position, as shown in Figure 5, and a stowed position, in which the articulating arm 104 recedes into a cavity 420 formed in the spacer panel 404. The UV lamp 106 may also be configured to slide or otherwise move relative to the second arm segment 412 in the direction of arrows A, such as via one or more tracks. Optionally, the UV lamp 106 may be fixed in relation to the second arm segment 412.

The articulating arm 104 allows the disinfecting system 100 to be moved to the deployed position, where the UV lamp 106 is closer to the disinfection zone 114, such as a breathing space of an individual 424 seated within the seat 406. As shown in Figure 5, the pivot joints 110a and 110b allow the articulating arm 104 to articulate relative to the X-Z plane.

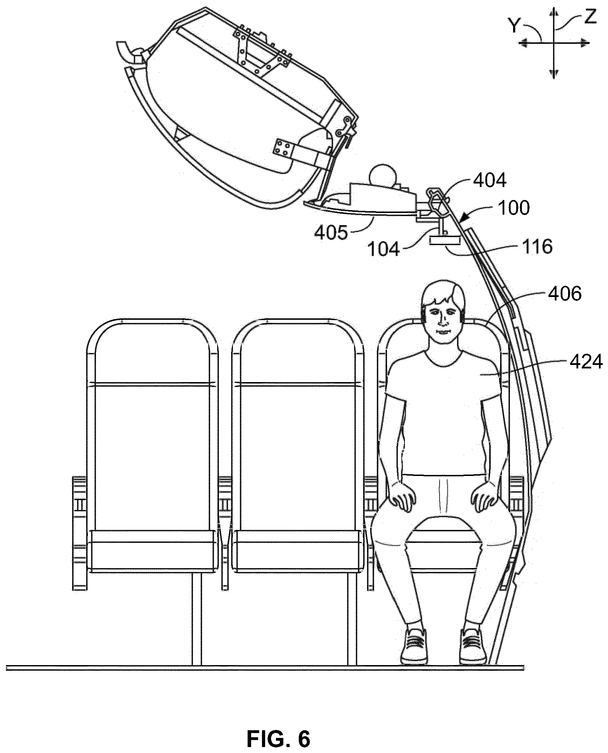

Figure 6 illustrates a front view of the disinfecting system 100 within the internal cabin of Figure 5. The spacer panel 404 is adjacent a PSU 405. Referring to Figures 5 and 6, the pivot joints 110a and 110b allow the articulating arm 104 to articulate relative to the Y-Z plane, as well as the X-Y plane, in addition to the X-Z plane.

The articulating arm 104 may include more or less pivot joints than those shown. For example, the articulating arm 104 may include a single arm segment coupled to the spacer panel 404 through a single pivot joint. As another example, the articulating 104 may include three arm segments coupled to one another through at least three pivot joints.

Referring to Figures 1, 5, and 6, the activation member 124 can be coupled to a portion of the seat 406 (such as an armrest), a portion of a PSU, and/or the like. Optionally, the system may not include the activation member 124.

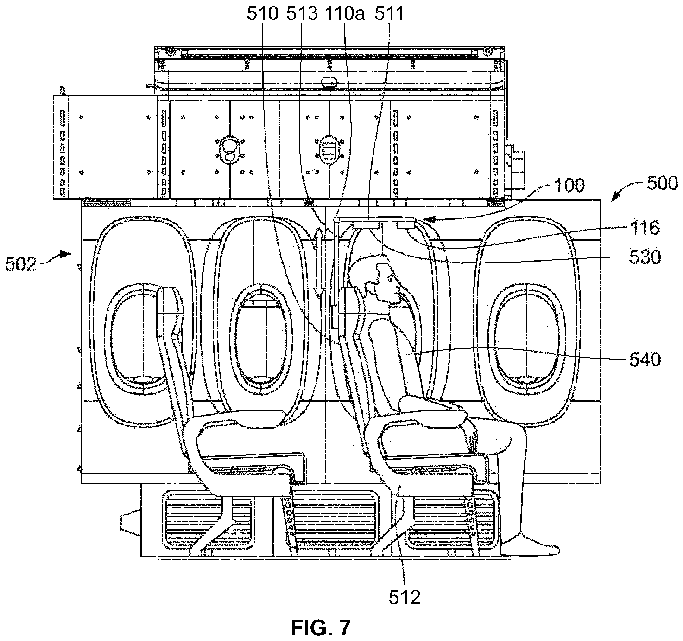

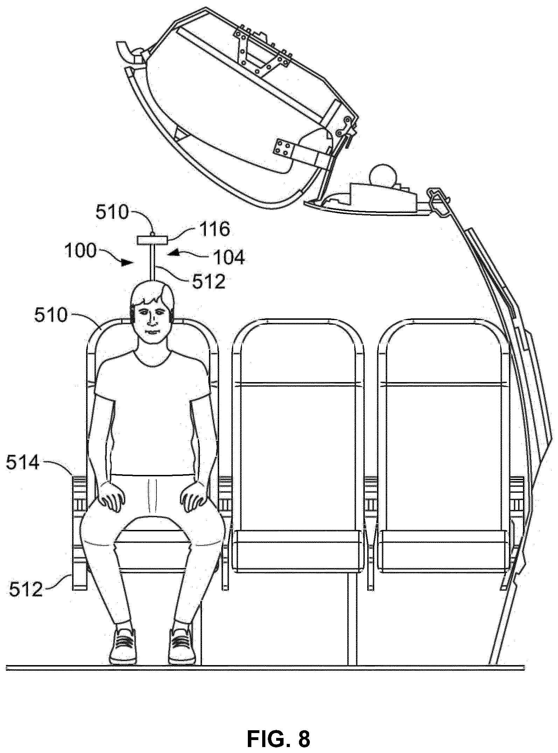

Figure 7 illustrates an example of a lateral view of a disinfecting system 100 within an internal cabin 500 of a vehicle 502. Figure 8 illustrates a front view of the disinfecting system 100 within the internal cabin 500 of Figure 7. Referring to Figures 7 and 8, in this example, the structure to which the disinfecting system 100 is moveably secured is a headrest 510 of a seat assembly 512. The articulating arm 104 includes a first arm segment 511 coupled to a second arm segment 513 through a first pivot joint 110a, which allows the first arm segment 511 and the second arm segment 513 to articulate relative to one another. The second arm segment 513 can moveably coupled to the headrest 510 through a second pivot joint, and/or a telescoping connection. In this manner, the articulating arm 104 can be moved between the deployed position, as shown in Figure 7, and a stowed position, in which the disinfecting system 100 is stowed behind the headrest 510.

In at least one example, the disinfecting system 100 also includes a distance ranging unit 530, such a laser sensor, an infrared sensor, an ultrasonic sensor, or the like. The distance ranging unit 530 is configured to detect an appropriate distance for the disinfecting system 100 relative to the individual 540. The distance ranging unit 530 can be in communication with the UV lamp 106, such as through one or more wired or wireless connections. In at least one example, the UV lamp 106 is configured to emit UV light in response to receiving a signal from the distance ranging unit 530 indicating that the UV lamp 106 is at a predetermined distance from the individual 540 associated with a predetermined appropriate distance. The distance ranging unit 530 can be used with any of the examples described herein. Optionally, the disinfecting system 100 may not include the distance ranging unit 530.

The disinfecting system 100 can be moveably secured to other portions of the seat assembly 512, such as to a lateral portion of the headrest 512, an armrest 514, or the like. Further, the articulating arm 104 may include more or less arm segments than shown.

Referring to Figures 1, 7, and 8, the activation member 124 can be coupled to a portion of the seat assembly 512 (such as an armrest), a portion of a PSU, and/or the like. Optionally, the system may not include the activation member 124.

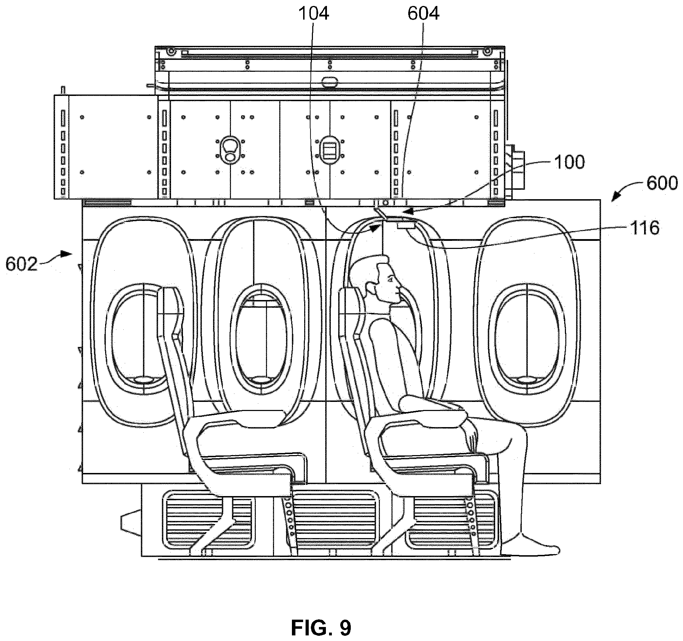

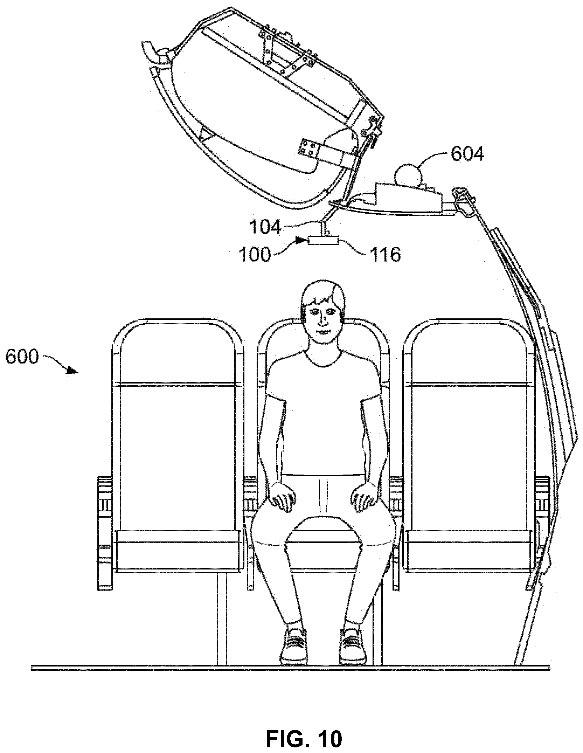

Figure 9 illustrates an example of a lateral view of a disinfecting system 100 within an internal cabin 600 of a vehicle 602. Figure 10 illustrates a front view of the disinfecting system 100 within the internal cabin 600 of Figure 9. Referring to Figures 9 and 10, the disinfecting system 100 is moveably coupled to a PSU 604 or a spacer panel connected thereto. The articulating arm 104 may include more or less arm segments than shown.

Referring to Figures 1, 9, and 10, the activation member 124 can be coupled to a portion of the seat (such as an armrest), a portion of a PSU, and/or the like. Optionally, the system may not include the activation member 124.

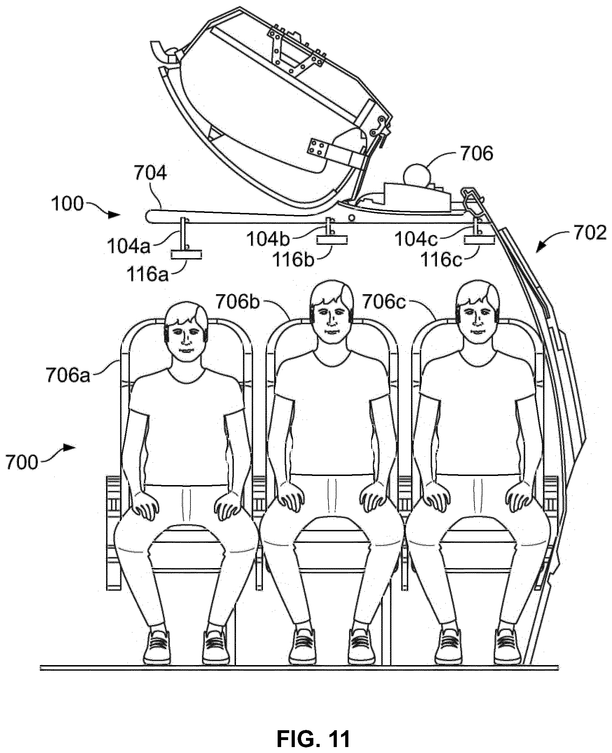

Figure 11 illustrates an example of a front view of a disinfecting system 100 within an internal cabin 700 of a vehicle 702. In this example, the disinfecting system 100 includes a support bar 704, which may be coupled to a structure, such as a PSU 706, a spacer panel connected thereto, and/or the like. The support bar 704 can be fixed to the structure, or moveable coupled thereto, such as through one or more pivot joints. A plurality of articulating arms 104a, 104b, and 104c such as any of those described herein are moveably coupled to the support bar 704, such as via one or more pivot joints. Each articulating arm 104a, 104b, and 104c is in turn coupled to a UV lamp 116a, 116b, and 116c, respectively. The support bar 704 and the articulating arms 104a-c can be moved to position the UV lamps 116a-116c over respective seats 706a, 706b, and 706c. More or less articulating arms and UV lamps can be coupled to the support bar 704.

Referring to Figures 1 and 11, the activation member 124 can be coupled to a portion of the seat (such as an armrest), a portion of a PSU, and/or the like. Optionally, the system may not include the activation member 124.

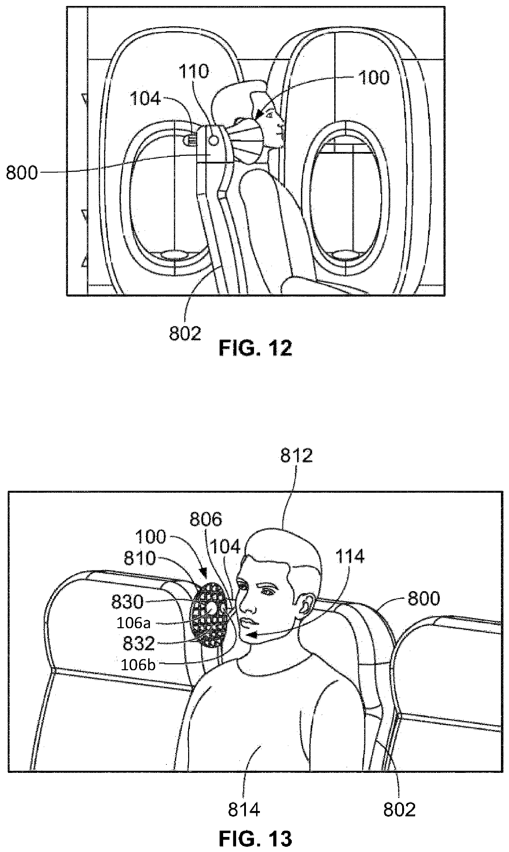

Figure 12 illustrates a lateral view of a disinfecting system 100 secured to a headrest 800 of a seat 802, according to an example of the claimed subject matter. Figure 13 illustrates a front perspective view of the disinfecting system 100 of Figure 12. The disinfecting system 100 includes the articulating arm 104 pivotally coupled to a side 806 of the headrest 800 via a pivot joint 110, which allows the articulating arm 104 to pivot between the deployed position shown in Figures 12 and 13 in which a main body 810 of the disinfecting system 100 is disposed to a side of a head 812 of an individual 814 seated within the seat 802, and a stowed position, in which the main body 810 is disposed between seats, behind and to a side of the seat, and/or the like.

In at least one example, the main body 810 includes the UV lamp 106, which is configured to emit the UV light into the disinfection zone 114, such as the breathing space of the individual 814. Optionally, the UV lamp 106 may be embedded within the main body 810 and configured to emit the UV light into an air stream passing through the main body 810, and which is expelled into the disinfection zone 114. In this manner, the UV lamp 106 does not directly emit the UV light into the disinfection zone 114, but rather within an air channel within the main body 810. An internal fan 830 may be within the main body 810, and is configured to blow the disinfected air stream out through air outlets 832 formed in the main body 810. According to the claimed subject matter, the disinfecting system 100 includes a first UV lamp 106a that is configured to emit the UV light into the disinfection zone 114, as well as an embedded second UV lamp 106b within the main body 810, which is configured to emit the UV light into an air stream within the main body 810. In at least one example, the disinfecting system 100 include one of the UV lamp 106a or the UV lamp 106b.

Referring to Figures 1, 12, and 13, the activation member 124 can be coupled to a portion of the seat (such as an armrest), a portion of a PSU, and/or the like. Optionally, the system may not include the activation member 124.

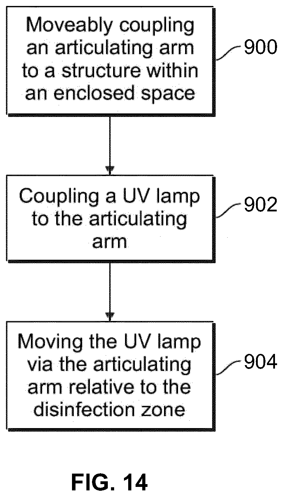

Figure 14 illustrates a flow chart of a disinfecting method. Referring to Figures 1 and 14, the disinfecting method is for the enclosed space 102, and includes moveably coupling, at 900, the articulating arm 104 to the structure 108 within the enclosed space 102; coupling, at 902, the UV lamp 106 to the articulating arm 104, wherein the UV lamp 106 is configured to emit the UV light 120 toward the disinfection zone 114 within the enclosed space 102; and moving, at 904, the UV lamp 106 via the articulating arm 104 relative to the disinfection zone 114. In at least one example, said moving 904 includes moving the UV lamp 106 between a stowed position and a deployed position.

In at least one example, the enclosed space 102 is an internal cabin of a vehicle. As a further example, the structure 108 is one or more of a spacer panel, a passenger service unit (PSU), a portion of a seat, or an overhead stowage bin assembly within the internal cabin.

As described herein, examples provide UV light disinfecting systems and methods that can be quickly and easily positioned with respect to an area, space or the like that is to be disinfected. Further, examples provide UV light disinfecting systems and methods that can be moveably secured within a confined space, such as within an internal cabin of a vehicle. Also, examples provide UV light disinfecting systems and methods that utilize a reduced amount of power, as the UV lamps can be moved into a close proximity with respect to a disinfection zone.

While various spatial and directional terms, such as top, bottom, lower, mid, lateral, horizontal, vertical, front and the like can be used to describe examples, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or aspects thereof) can be used in combination with each other. In addition, many modifications can be made to adapt a particular situation or material to the teachings of the various examples without departing from their scope. While the dimensions and types of materials described herein are intended to define the parameters of the various examples, the examples are by no means limiting and are exemplary examples. Many other examples will be apparent to those of skill in the art upon reviewing the above description. The scope of the various examples should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various examples to enable any person skilled in the art to practice the various examples, including making and using any devices or systems and performing any incorporated methods. The patentable scope is defined by the claims, and can include other examples that occur to those skilled in the art. Such other examples are intended to be within the scope of the claims if the examples have structural elements that do not differ from the literal language of the claims, or if the examples include equivalent structural elements with insubstantial differences from the literal language of the claims.

## Claims

1. A disinfecting system (100) for an enclosed space (102), the disinfecting system (100) comprising:
a seat (802) with a headrest (800), wherein the headrest (800) comprises a pivot joint (110) at a side (806);
an articulating arm (104) pivotally coupled to the side (806) of the headrest (800) via the pivot joint (110) within the enclosed space (102);
a main body (810) coupled to the articulating arm (104) and comprising a first and second ultraviolet (UV) lamp (106a, 106b), wherein the articulating arm (104) is configured to pivot between a deployed position in which the main body (810) is disposed to a side of a head (812) of an individual (814) seated within the seat (802), and a stowed position in which the main body (810) is behind and to a side of the seat (802),
wherein the first UV lamp (106a) is configured to emit UV light (120) toward a disinfection zone (114) within the enclosed space (102), and
wherein the second ultraviolet (UV) lamp (106b) is embedded within the main body (810) and configured to emit the UV light (120) into an air stream passing through the main body (810),
wherein the articulating arm (104) allows the UV lamps (106a, 106b) to move relative to the disinfection zone (114).

2. The disinfecting system (100) of claim 1, further comprising an internal fan (830) within the main body (810), wherein the internal fan (830) is configured to blow the disinfected air stream out through air outlets (832) formed in the main body (810), such that the second UV lamp (106b) is configured to emit the UV light (120) into the air stream within the main body (810).

3. The disinfecting system (100) of claim 1 or 2, wherein the articulating arm (104) comprises one or more pivot joints (110).

4. The disinfecting system (100) of claim 3, further comprising one or more dampers (112) coupled to the one or more pivot joints (110).

5. The disinfecting system (100) of claim 1, further comprising a latch (122) coupled to the headrest (800) of the seat (802) and the articulating arm (104), wherein the latch (122) is selectively moveable between a locked position and an unlocked position.

6. The disinfecting system (100) of claim 5, further comprising an activation member (124) that is configured to control the latch (122).

7. The disinfecting system (100) of claim 6, further comprising a switch (126), wherein the activation member (124) is coupled to the switch (126), which is in turn coupled to the latch (122).

8. The disinfecting system (100) of claim 7, wherein the switch (126) is configured to move from a locked position to an unlocked position, thereby allowing the disinfecting system (100) to move from the stowed position into the deployed position.

9. The disinfecting system (100) of any one of claims 6 to 8, the seat (802) further comprising an armrest, wherein the activation member (124) is coupled to the armrest of the seat (802).

10. The disinfecting system (100) of any one of claims 6 to 8, further comprising a passenger service unit, wherein the activation member (124) is coupled to a portion of the passenger service unit.

11. The disinfecting system (100) of any one of claims 6 to 10, wherein the activation member (124) is configured to be engaged by the individual (814).

12. The disinfecting system (100) of claim 1, wherein the enclosed space (102) is an internal cabin of a vehicle.

13. The disinfecting system (100) of claim 1, wherein the first UV lamp (106a) is configured to emit the UV light (120) into the disinfection zone (114) including a breathing space of the individual (814) seated within the seat (802).

14. The disinfecting system (100) of claim 1, further comprising a distance ranging unit (530).

15. The disinfecting system (100) of claim 14, wherein the distance ranging unit (530) is one of a laser sensor, an infrared sensor, or an ultrasonic sensor.

## Patentansprüche

1. Desinfektionssystem (100) für einen geschlossenen Raum (102), wobei das Desinfektionssystem (100) aufweist:
einen Sitz (802) mit einer Kopfstütze (800), wobei die Kopfstütze (800) ein Drehgelenk (110) an einer Seite (806) aufweist;
einen Gelenkarm (104), der über das Drehgelenk (110) innerhalb des geschlossenen Raums (102) schwenkbar mit der Seite (806) der Kopfstütze (800) gekoppelt ist;
einen Hauptkörper (810), der mit dem Gelenkarm (104) gekoppelt ist und eine erste und eine zweite Ultraviolett-(UV)-Lampe (106a, 106b) aufweist, wobei der Gelenkarm (104) konfiguriert ist, zwischen einer ausgefahrenen Position, in der der Hauptkörper (810) an einer Seite eines Kopfes (812) einer Person (814), die in dem Sitz (802) sitzt, angeordnet ist, und einer verstauten Position, in der sich der Hauptkörper (810) hinter und an einer Seite des Sitzes (802) befindet, zu schwenken,
wobei die erste UV-Lampe (106a) konfiguriert ist, UV-Licht (120) zu einer Desinfektionszone (114) innerhalb des geschlossenen Raums (102) zu emittieren, und
wobei die zweite Ultraviolett-(UV)-Lampe (106b) in den Hauptkörper (810) eingebettet und konfiguriert ist, das UV-Licht (120) in einen durch den Hauptkörper (810) strömenden Luftstrom zu emittieren,
wobei der Gelenkarm (104) es ermöglicht, dass sich die UV-Lampen (106a, 106b) relativ zur Desinfektionszone (114) bewegen.

2. Desinfektionssystem (100) nach Anspruch 1, das ferner einen internen Lüfter (830) im Hauptkörper (810) aufweist, wobei der interne Lüfter (830) konfiguriert ist, den desinfizierten Luftstrom durch Luftauslässe (832), die im Hauptkörper (810) ausgebildet sind, so zu blasen, dass die zweite UV-Lampe (106b) konfiguriert ist, das UV-Licht (120) in den Luftstrom im Hauptkörper (810) zu emittieren.

3. Desinfektionssystem (100) nach Anspruch 1 oder 2, wobei der Gelenkarm (104) ein oder mehrere Drehgelenke (110) aufweist.

4. Desinfektionssystem (100) nach Anspruch 3, das ferner eine oder mehrere Dämpfungen (112) aufweist, die mit dem einen oder den mehreren Drehgelenken (110) gekoppelt sind.

5. Desinfektionssystem (100) nach Anspruch 1, das ferner eine Verriegelung (122) aufweist, die mit der Kopfstütze (800) des Sitzes (802) und dem Gelenkarm (104) gekoppelt ist, wobei die Verriegelung (122) selektiv zwischen einer verriegelten Position und einer entriegelten Position beweglich ist.

6. Desinfektionssystem (100) nach Anspruch 5, das ferner ein Aktivierungselement (124) aufweist, das konfiguriert ist, die Verriegelung (122) zu steuern.

7. Desinfektionssystem (100) nach Anspruch 6, das ferner einen Schalter (126) aufweist, wobei das Aktivierungselement (124) mit dem Schalter (126) gekoppelt ist, der wiederum mit der Verriegelung (122) gekoppelt ist.

8. Desinfektionssystem (100) nach Anspruch 7, wobei der Schalter (126) konfiguriert ist, sich von einer verriegelten Position in eine entriegelte Position zu bewegen, wodurch es ermöglicht wird, dass sich das Desinfektionssystem (100) von der verstauten Position in die ausgefahrene Position bewegt.

9. Desinfektionssystem (100) nach einem der Ansprüche 6 bis 8, wobei der Sitz (802) ferner eine Armlehne aufweist, wobei das Aktivierungselement (124) mit der Armlehne des Sitzes (802) gekoppelt ist.

10. Desinfektionssystem (100) nach einem der Ansprüche 6 bis 8, das ferner eine Passagierserviceeinheit aufweist, wobei das Aktivierungselement (124) mit einem Teil der Passagierserviceeinheit gekoppelt ist.

11. Desinfektionssystem (100) nach einem der Ansprüche 6 bis 10, wobei das Aktivierungselement (124) so konfiguriert ist, dass es von der Person (814) eingeschaltet werden kann.

12. Desinfektionssystem (100) nach Anspruch 1, wobei der geschlossene Raum (102) eine Innenkabine eines Fahrzeugs ist.

13. Desinfektionssystem (100) nach Anspruch 1, wobei die erste UV-Lampe (106a) konfiguriert ist, das UV-Licht (120) in die Desinfektionszone (114) zu emittieren, die einen Atemraum der Person (814) aufweist, die auf dem Sitz (802) sitzt.

14. Desinfektionssystem (100) nach Anspruch 1, das ferner eine Abstandsmesseinheit (530) aufweist.

15. Desinfektionssystem (100) nach Anspruch 14, wobei die Abstandsmesseinheit (530) ein Lasersensor, ein Infrarotsensor oder ein Ultraschallsensor ist.

## Revendications

1. Système de désinfection (100) pour un espace clos (102), le système de désinfection (100) comprenant :
un siège (802) avec un appuie-tête (800), dans lequel l'appuie-tête (800) comprend une articulation pivot (110) d'un côté (806) ;
un bras articulé (104) accouplé de manière pivotante au côté (806) de l'appuie-tête (800) via l'articulation pivot (110) dans l'espace clos (102) ;
un corps principal (810) accouplé au bras articulé (104) et comprenant une première et une seconde lampe à ultraviolets (UV) (106a, 106b), dans lequel le bras articulé (104) est configuré pour pivoter entre une position déployée dans laquelle le corps principal (810) est disposé sur un côté d'une tête (812) d'un individu (814) assis dans le siège (802), et une position rangée dans laquelle le corps principal (810) est derrière et sur un côté du siège (802),
dans lequel la première lampe UV (106a) est configurée pour émettre de la lumière UV (120) vers une zone de désinfection (114) dans l'espace clos (102), et
dans lequel la seconde lampe à ultraviolets (UV) (106b) est intégrée dans le corps principal (810) et configurée pour émettre la lumière UV (120) dans un flux d'air passant à travers le corps principal (810),
dans lequel le bras articulé (104) permet aux lampes UV (106a, 106b) de se déplacer par rapport à la zone de désinfection (114).

2. Système de désinfection (100) selon la revendication 1, comprenant en outre un ventilateur interne (830) dans le corps principal (810), dans lequel le ventilateur interne (830) est configuré pour souffler le flux d'air désinfecté à travers des sorties d'air (832) formées dans le corps principal (810), de sorte que la seconde lampe UV (106b) soit configurée pour émettre la lumière UV (120) dans le flux d'air dans le corps principal (810).

3. Système de désinfection (100) selon la revendication 1 ou 2, dans lequel le bras articulé (104) comprend une ou plusieurs articulations pivots (110).

4. Système de désinfection (100) selon la revendication 3, comprenant en outre un ou plusieurs amortisseurs (112) accouplés aux une ou plusieurs articulations pivots (110).

5. Système de désinfection (100) selon la revendication 1, comprenant en outre un loquet (122) accouplé à l'appuie-tête (800) du siège (802) et au bras articulé (104), dans lequel le loquet (122) est mobile sélectivement entre une position verrouillée et une position déverrouillée.

6. Système de désinfection (100) selon la revendication 5, comprenant en outre un élément d'activation (124) qui est configuré pour commander le loquet (122).

7. Système de désinfection (100) selon la revendication 6, comprenant en outre un commutateur (126), dans lequel l'élément d'activation (124) est accouplé au commutateur (126), qui est à son tour accouplé au loquet (122).

8. Système de désinfection (100) selon la revendication 7, dans lequel le commutateur (126) est configuré pour passer d'une position verrouillée à une position déverrouillée, permettant ainsi au système de désinfection (100) de passer de la position rangée à la position déployée.

9. Système de désinfection (100) selon l'une quelconque des revendications 6 à 8, le siège (802) comprenant en outre un accoudoir, dans lequel l'élément d'activation (124) est accouplé à l'accoudoir du siège (802).

10. Système de désinfection (100) selon l'une quelconque des revendications 6 à 8, comprenant en outre un bloc service passagers, dans lequel l'élément d'activation (124) est accouplé à une partie du bloc service passagers.

11. Système de désinfection (100) selon l'une quelconque des revendications 6 à 10, dans lequel l'élément d'activation (124) est configuré pour être actionné par l'individu (814).

12. Système de désinfection (100) selon la revendication 1, dans lequel l'espace clos (102) est une cabine interne d'un véhicule.

13. Système de désinfection (100) selon la revendication 1, dans lequel la première lampe UV (106a) est configurée pour émettre la lumière UV (120) dans la zone de désinfection (114) comprenant un espace de respiration de l'individu (814) assis dans le siège (802).

14. Système de désinfection (100) selon la revendication 1, comprenant en outre une unité de mesure de distance (530).

15. Système de désinfection (100) selon la revendication 14, dans lequel l'unité de mesure de distance (530) est un parmi un capteur laser, un capteur infrarouge ou un capteur ultrasonore.
